(19) Europäisches Patentamt
European Patent Office
Office européen des brevets

(11) EP 1 285 907 B1

(12) **EUROPÄISCHE PATENTSCHRIFT**

(45) Veröffentlichungstag und Bekanntmachung des Hinweises auf die Patenterteilung:
**19.10.2011 Patentblatt 2011/42**

(21) Anmeldenummer: **02017800.0**

(22) Anmeldetag: **13.08.2002**

(51) Int Cl.:
*C07C 69/28* (2006.01) *C07C 69/16* (2006.01)
*A61K 8/33* (2006.01) *A61K 8/40* (2006.01)
*A61Q 5/10* (2006.01) *A61Q 5/12* (2006.01)
*C11D 3/50* (2006.01)

(54) **Depotpräparate zur gezielten Freisetzung von einem Aldehyd zusammen mit zwei Carbonsäuren**

Compositions for the controlled release of an aldehyde together with two carboxylic acids

Compositions à libération contrôlée d'un aldéhyde conjointement avec deux acides carboxyliques

(84) Benannte Vertragsstaaten:
**AT BE BG CH CY CZ DE DK EE ES FI FR GB GR IE IT LI LU MC NL PT SE SK TR**

(30) Priorität: **20.08.2001 DE 10140786**

(43) Veröffentlichungstag der Anmeldung:
**26.02.2003 Patentblatt 2003/09**

(73) Patentinhaber: **Symrise AG**
**37603 Holzminden (DE)**

(72) Erfinder:
- **Eh, Marcus Dr.**
**37603 Holzminden (DE)**
- **Surburg, Horst Dr.**
**37603 Holzminden (DE)**
- **Sonnenberg, Steffen Dr.**
**37603 Holzminden (DE)**

(74) Vertreter: **Eisenführ, Speiser & Partner**
**Am Kaffee-Quartier 3**
**28217 Bremen (DE)**

(56) Entgegenhaltungen:
**EP-A- 0 255 904** **EP-A- 0 456 932**
**EP-A- 0 963 974** **WO-A-00/04009**
**WO-A-98/50009** **DE-A- 3 729 074**
**DE-B- 1 145 161** **US-A- 4 395 430**

- **DATABASE CA [Online] CHEMICAL ABSTRACTS SERVICE, COLUMBUS, OHIO, US; KINOSHITA, SHOICHI ET AL: "Preparation of .alpha.,.beta.-unsaturated aldhydes" retrieved from STN Database accession no. 108:56379 XP002230701 & JP 62 021772 A (DAINIPPON INK UND CHEMICALS ) 30. Januar 1987 (1987-01-30)**
- **DATABASE CA [Online] CHEMICAL ABSTRACTS SERVICE, COLUMBUS, OHIO, US; ZHANG, LING ET AL.: "Constituents of essential oils in lonicera japonica" retrieved from STN Database accession no. 122:89050 XP002230702 & ZHONGGUO YAOKE DAXUE XUEBAO, Bd. 25, Nr. 3, 1994, Seiten 184-7,**
- **DATABASE CROSSFIRE BEILSTEIN [Online] Beilstein Institut zur Förderung der Chemischen Wissenschaften, Frankfurt am Main, DE; Database accession no. BRN 1874512 XP002230703 & OGIBIN, YU. N. ET AL.: BULL. ACAD. SCI. USSR DIV. CHEM. SCI. (ENGL. TRANSL.), 1965, Seiten 361-3,**
- **DATABASE CROSSFIRE BEILSTEIN [Online] Beilstein Institut zur Förderung der Chemischen Wissenschaften, Frankfurt am Main, DE; Database accession no. RID 557101 XP002230704 & WAKAYAMA: NIPPON KAGAKU ZASSHI; 72; 1951; 275,**
- **DATABASE CROSSFIRE BEILSTEIN [Online] Beilstein Institut zur Förderung der Chemischen Wissenschaften, Frankfurt am Main, DE; Database accession no. BRN 2656096 XP002230705 & HECK, R. F.: ORGANOMET. CHEM. SYNTH., 1; 1972; 455-62,**
- **DATABASE CROSSFIRE BEILSTEIN [Online] Beilstein Institut zur Förderung der Chemischen Wissenschaften, Frankfurt am Main, DE; Database accession no. RID 250041 XP002230706 & SEMMLER: CHEM. BER.; 42; 1909; 1161,**
- **DATABASE CROSSFIRE BEILSTEIN [Online] Beilstein Institut zur Förderung der Chemischen Wissenschaften, Frankfurt am Main, DE; Database accession no. BRN 1984733 XP002230707 & SUMIDA, NORIHIKO ET AL.: SYN. LETT.; 12; 1921-22,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **DATABASE CROSSFIRE BEILSTEIN [Online] Beilstein Institut zur Förderung der Chemischen Wissenschaften, Frankfurt am Main, DE; Database accession no. BRN 1976711 XP002230708 & WEGSCHEIDER; SPAETH: MONATSH. CHEM.; 30; 1909; 841,**

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft permanente Haarfärbemittel enthaltend Depotpräparate ("Delivery Systems") zur gezielten Freisetzung von einem Aldehyd zusammen mit zwei Carbonsäuren. Bei den freigesetzten Aldehyden handelt es sich um organoleptische Substanzen, im speziellen um Riechstoffe oder Geschmackstoffe. Die Depotpräparate sind erhältlich durch Umsetzung von Aldehyden mit Carbonsäureanhydriden.

Die prinzipielle Vorgehensweise zur Parfümierung von Konsumartikeln ist die, dass das Riechstoffe enthaltene Parfümöl direkt mit dem Produkt vermischt wird. Die Probleme die hierbei auftreten sind, dass leichtflüchtige Substanzen während der Einarbeitung in das Produkt oder im Verlauf der Lagerung teilweise oder vollständig durch Ausdampfen verloren gehen. Weiterhin sind zahlreiche Substanzen und hierbei besonders auch Aldehyde instabil unter den gegebenen Bedingungen, was zur teilweisen oder vollständigen Zersetzung dieser Moleküle führt. Die Konsequenz hieraus ist, dass alle Substanzen, die den oben beschriebenen Problemen unterliegen im Endprodukt sensorisch nur noch schwach oder gar nicht mehr wahrnehmbar sind. Dies kann in Einzelfällen zu einer Veränderung des Gesamtgeruchseindrucks der Komposition führen.

Aus WO 94/06441 sind Acetale, Ketale und spezielle Orthoester als im basischen stabile Depotpräparate bekannt. Im sauren Milieu erfolgt, wie z.B. im Kontakt mit der Haut, die Hydrolyse, so dass Alkohole und Ketone freigesetzt werden.

Ein Depotpräparat zur Freisetzung von Aldehyden, im speziellen Citral, in Lebensmitteln und hier im speziellen in alkoholischen oder nicht-alkoholischen Getränken wird in WO 00/04009 beschrieben. Hierbei handelt es sich um Dicarboalkoxydioxolane, welche durch Acetalisierung aus einem Aldehyd und einem Weinsäurederivat zugänglich sind. Sie besitzen in wässrig sauren alkoholischen und nicht-alkoholischen Getränken eine sehr viel höhere Halbwertszeit als vergleichbare Acetale.

WO 00/38616 beansprucht ein zyklisches Dioxaketon, welches nach Hydrolyse gleichzeitig ein Aldehyd oder Keton und eine Hydroxycarbonsäure freisetzt. Die Herstellung dieser Verbindungen gelingt durch Umsetzung der entsprechenden Hydroxycarbonsäure mit dem Aldehyd oder Keton unter Zugabe katalytischer Mengen Säure am Wasserabscheider. Bevorzugt sind Aldehyde oder Ketone mit Riechstoffeigenschaften und $\alpha$-Hydroxycarbonsäuren oder ringsubstituierteBenzoesäuren.

Database CA [Online] Chemical Abstracts Service, accession no. 108:56379 offenbart Verbindungen der unten aufgeführten Formel I in bestimmten Anwendungen.

Für keines der oben beschriebenen Depotpräparate sind jedoch die Fähigkeit simultan die Riechstofikomponente neben zwei weiteren Verbindungen in permanenten Haarfärbemitteln bei guter Lagerstabilität schnell freizusetzen aus dem Stand der Technik bekannt. Weiterhin werden die Probleme, die bei der konventionellen Parfümierung von Konsumartikeln auftreten (Ausdampfen während der Lagerung, Instabilität usw.), durch das erfindungsgemäß einzusetzende Depotpräparat gelöst.

Gegenstand der Erfindung ist ein permanentes Haarfärbemittel, enthaltend Riechstoffe oder Parfümöl, wobei die Riechstoffe oder das Parfümöl ein Depotpräparat Formel (I)

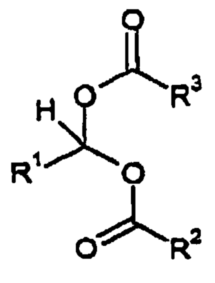

(I)

enthalten, worin

$R^1$, $R^2$ und $R^3$ - unabhängig voneinander - einen organischen Rest mit 1 bis 30 Kohlenstoffatomen bedeuten und die Verbindung der Formel (I) nach Hydrolyse oder enzymatischer Spaltung einen Aldehyd und zwei Carbonsäuren freisetzt.

In einer bevorzugten Ausführungsform der Erfindung werden Verbindungen der Formel (I) eingesetzt, in denen

$R^1$ für einen gesättigten oder ungesättigten, linearen oder verzweigten, alicyclischen oder aromatischen Rest mit 1 bis 18 Kohlenstoffatomen steht, der gegebenenfalls noch Heteroatome enthalten kann,

$R^2$ und $R^3$ unabhängig voneinander für einen gesättigten oder ungesättigten, linearen oder verzweigten, alicyclischen oder aromatischen Rest mit 1 bis 22 Kohlenstoffatomen stehen, die gegebenenfalls noch Heteroatome enthalten können und die gegebenenfalls mit ionischen Substituenten substituiert sein können,

oder worin

$R^2$ und $R^3$ zusammen einen gesättigten oder ungesättigten, verzweigten oder unverzweigten carbocyclischen oder aromatischen Ring aus 3 bis 10 Kohlenstoff atomen, bevorzugt aus 4 bis 8 Kohlenstoffatomen, bilden.

Bevorzugte Heteroatome sind Sauerstoff und Schwefel. Besonders bevorzugt ist Sauerstoff.

Beispiele für ionische Substituenten sind $-CO_2M$ oder $-OCO_2M$, wobei M ein Alkalimetall ist.

Die erfindungsgemäß einzusetzenden Acylale der Formel (I) zerfallen einerseits nach wässriger Hydrolyse, bevorzugt in alkalisch wässrigem Medium mit einem pH $\geq$ 8 wie auch in sauer wässrigem Medium mit einem pH < 3, besonders bevorzugt ist für die alkalische Hydrolyse ein pH Bereich von 9 bis 13 und für die saure Hydrolyse ein pH

Bereich von 0 bis 2,5, so dass ein Aldehyd und zwei Carbonsäuren freigesetzt werden, die dann ihre Wirkung (z.B. Duftfreisetzung) entfalten und sich an Substrate anheften können. Andererseits zerfallen die erfindungsgemäß einzusetzenden Acylale der Formel (I) nach enzymatischer Spaltung, so dass ein Aldehyd und zwei Carbonsäuren freigesetzt werden, die dann ihre Wirkung (z.B. Duftfreisetzung) entfalten und sich an Substrate anheften können.

Überraschenderweise lässt sich die Freisetzungsrate über die Reste $R^2$ und $R^3$, in Abhängigkeit von $R^1$, in der Art steuern, dass Acylale mit kleinen und schmalen Resten $R^2$ und $R^3$ kurze Halbwertszeiten besitzen, wohingegen voluminöse und langkettige Reste $R^2$ und $R^3$ die Halbwertszeit der Acylale erhöhen. Dies ist insofern von Bedeutung, da somit Acylale der Formel (I) mit unterschiedlichen Freisetzungsprofilen hergestellt werden können. Darauf aufbauend können das Depotpräparat und die Formulierung in ihren jeweiligen Resten bzw. Inhaltsstoffen aufeinander abgestimmt werden, um durch kontrollierte Freisetzung bzw. kontrollierte Haftung/Übertragung eine applikationsorientierte Freisetzung zu erzielen.

Die enzymatische Spaltung kann bevorzugt durch Esterasen oder Lipasen durchgeführt werden.

In einer weiteren bevorzugten Ausführungsform der Erfindung hat der aus der Verbindung der Formel (I) freigesetzte Aldehyd ein Molekulargewicht von 100 bis 350 g/mol und besonders bevorzugt von 120 bis 270 g/mol. Weiterhin ist bevorzugt, wenn der freigesetzte Aldehyd ein Riechstoff oder Gesehmackstoffist.

Nicht limitierende Beispiele für Aldehyde, die nach Spaltung des erfindungsgemäß einzusetzenden Depotpräparates freigesetzt werden können, sind im folgenden genannt:

Phenylacetaldehyd, p-Methylphenylacetaldehyd, p-Isopropylphenylacetaldehyd, Methylnonyl acetaldehyd, phenylpropanal, 3-(4-t-Butylphenyl)-2-methylpropanal (Lilial), 3-(4-t-Butylphenyl)-propanal (Bourgeonal), 3-(4-Methoxyphenyl)-2-methylpropanal (Canthoxal), 3-(4- Isopropylphenyl)-2-methylpropanal (Cymal), 3-(3,4-Methylendioxyphenyl)-2-methylpropanal (Helional), 3-(4-Ethylphenyl)-2,2-dimethylpropanal (Floralozone), Phenylbutanal, 3-Methyl-5-phenylpentanal, Hexanal, trans-2-Hexenal, cis-Hex-3-enal, Heptanal, cis-4-Heptenal, 2-Ethyl-2-heptenal, 2,6-Dimethyl-5-heptenal (Melonal), 2,4-Heptadienal, Octanal, 2-Octenal, 3,7-Dimethyloctanal, 3,7-Dimethyl-2,6-octadien-1-al, 3,7-Dimethyl-2,6-octadien-3-al, 3,7-Dimethyl-6-octenal (Citronellal), 3,7-Dimethyl-7-hydroxyoctan-1-al (Hydroxy Citronellal), Nonanal, 6-Nonenal, 2,4-Nonadienal, 2,6-Nonadienal, Decanal, 2-Methyldecanal, 4-Decenal, 9-Decenal, 2,4-Decadienal, Undecanal, 2-Methyldecanal, 2-Methylundecanal, 2,6,10-Trimethyl-9-undecenal (Adoxal), Undec-10-enylaldehyd, Undec-8-enanal, Dodecanal, Tridecanal, Tetradecanal, Anisaldehyd, Zimtaldehyd, $\alpha$-Amylzimtaldehyd, $\alpha$-Hexylzimtaldehyd, Methoxyzimtaldehyd, Isocyclocitral,Citronellyloxyacetaldehyd, Cortexaldehyd, Cuminaldehyd, Cyclamenaldehyd, Florhydral, Heliotropin, Hydratropaldehyd, Vanillin, Ethylvanillin, Benzaldehyd, p-Methylbenzaldehyd, 3,4-Dimethoxybenzaldehyd, 3- und 4-(4-Hydroxy-4-methylpentyl)-3-cyclohexen-1-carboxaldehyd (Lyral), 2,4-Dimethyl-3-cyclohexen-1-carboxaldehyd (Triplal), 1-Methyl-3-(4-methylpentyl)-3-cyclohexencarboxaldehyd (Vernaldehyd) oder p-Methylphenoxyacetaldehyd (Xi aldehyd) ist.

Nicht limitierende Beispiele für Carbonsäuren, die nach Spaltung des erfindungsgemäß einzusetzenden Depotpräparates freigesetzt werden können, sind im folgenden genannt:

Unsubstituierte, gesättigte Monocarbonsäuren (z.B. Essigsäure, Propionsäure, Buttersäure, Valeriansäure, Capronsäure, Caprylsäure, Pelargonsäure, Caprinsäure, Laurinsäure, Myristinsäure, Palmitinsäure, Stearinsäure); dialkylsubstituierte Essigsäuren (z.B. 2-Butyloctansäure, 2-Butyldecansäure, 2-Hexyloctansäure, 2-Hexyldecansäure); einfach oder mehrfach ungesättigte Monocarbonsäuren (z.B. Ölsäure, Linolsäure, $\alpha$-Linolensäure, $\gamma$-Linolensäure); Alkin-, Alkadiin-, oder Alkatriincarbonsäuren (z.B. Non-8-insäure, Dec-9-insäure, Tridec-9-insäure,13-Methyltetradec-9-insäure, Pentadec-6-insäure, Pentadec-7-insäure, Hexadec-9-insäure, 15-Methylhexadec-9-insäure, Heptadec-2-insäure, Heptadec-9-insäure, Octadec-12-insäure, Octadec-6,12-diinsäure, Nonadec-9-insäure); $\alpha$-Hydroxy-

carbonsäuren (z.B. α-Hydroxyvaleriansäure, α-Hydroxycapronsäure, α-Hydroxycaprylsäure, α-Hydroxypelargonsäure, α-Hydroxycaprinsäure, α-Hydroxylaurinsäure, α-Hydroxymyristinsäure, α-Hydroxypalmitinsäure, α-Hydroxypaimitinsäure); unsubstituierte α,ω-Alkandicarbonsäuren (z.B. Malonsäure, Bernsteinsäure, Glutarsäure, Adipinsäure, Pimelinsäure, Korksäure, Azelainsäure).

Eine weitere bevorzugte Ausführungsform des erfindungsgemäß einzusetzenden Depotpräparates beinhaltet Acylale der Formel (I), welche eine geringe Löslichkeit in Wasser besitzen und somit eine relativ große Tendenz haben sich an Substrate anzuheften, bzw. sich im Gasraum über einer wässrigen Lösung anzureichern. Wünschenswert ist hierbei, dass die erfindungsgemäß einzusetzenden Acylale der Formel (I) eine geringere Wasserlöslichkeit als die freiwerdenden Carbonsäuren haben, so dass eine bessere Anheftung auch der Carbonsäuren an das Substrat erfolgen kann, bzw. eine Anreicherung im Gasraum über der wässrigen Phase stattfindet. Löslichkeiten können direkt gemessen oder einfacher durch Heranziehung von Octanol/Wasser Verteilungskoeffizienten (log P Wert) bestimmt werden. Der log P Wert ist in der Literatur (A. Leo, C. Hansch und D. Elkins, Chem. Rev., 71, 1971, 525-616; C. Hansch, J.E. Quinlan, G.L. Lawrence, J. Org. Chem., 33, 1968, 347-350) eine etablierte Größe zur Bestimmung der Lipophilie.

Für Moleküle mit einem niedrigen log P Wert ist der Übergang aus einem wässrigen System an ein Substrat sehr schwierig, vielmehr haben solche Moleküle die Tendenz sich zu lösen und weggespült zu werden. Dies trifft insbesondere auf saure Verbindungen zu. Die vorliegende Erfindung kann diese Problematik beheben, da der Übergang der Carbonsäure mit einem niedrigen log P Wert aus der wässrigen Lösung an ein Substrat in der chemisch gebundenen Form der erfindungsgemäß einzusetzenden Acetale der Formel (I) mit einem höheren log P Wert erfolgt. Das bedeutet, dass die Übertragung einer Carbonsäure nach Freisetzung aus dem Depotpräparat mit einer höheren Rate erfolgt als die Übertragung der freien Carbonsäure. Die Freisetzung erfolgt nach anschließender Spaltung des erfindungsgemäß einzusetzenden Depotpräparates.

Die kontrollierte Freisetzung des Aldehyds und der zwei Carbonsäuren aus dem erfindungsgemäß einzusetzenden Depotpräparat kann zur Behandlung (z.B. Beduftung) von einer Fülle an Substraten, wie z.B. Haare, menschliche Haut, Wäsche und harte Oberflächen, benutzt werden.

Beispiele für Riechstoffe oder Parf-umöle, mit denen sich das erfindungs gemäß einzusetzende Depotpräparat vorteilhaft kombinieren lässt, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3rd. Ed., Wiley-VCH, Weinheim 1997.

Im einzelnen seien genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepresst; Linaloeöl; Litsea-cubeba-Öl; Lor-Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;

der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;

der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;

der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;

der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;

der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;

der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;

der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;

der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-teü,amethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);

der cyclischen Alkohole wie z.B. 4-tert.-ButylcycJohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;

der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-y1)-2-buten-1-ol; 2-Ethyl-4-(2,2,3-trimethyl-3-çyclopent-1-y1)-2-buten-l-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;

der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;

der cyclischen Ketone wie z.B. 4-tert.-Butylcyclohexanon; 2,2,5- Trimethyl-5-pentylcyclopentanon; 2-Heptylcyclopentanon; 2-Pentylcyclopentanon; 2-Hydroxy-3-methyl-2-cyclopenten-l-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;

der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4.-methylpentyl)-3-cyclohexencarbaldehyd; 4-(4-Methyl-3-penten-1-yl)-3-cyclohexencarbaldehyd;

der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;

der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;

der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;

der aromatischen Kohlenwasserstoffe wie z. B. Styrol und Diphenylmethan;

der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;

der Ester von araliphatischen Alkoholen mit aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether, Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno [1,2-d]-m-dioxin; 4,4a,5,9b-Tetrahydro-2,4-dimethylindeno[1,2-d]-m-dioxin;

der aromatischen oder araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;

der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;

der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;

der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;

der Phenole, Phenylether oder Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylpheaol; 2-Ethoxy-5-(1-propenyl)phenol; p-Kresylphenylacetat;

der heterocyclischen Verbindungen wie z.B. 2,5-Dirüethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;

der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Riechstoffe oder Parfümöle, die das erfindungsgemäß einzusetzende Depotpräparat enthalten, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.
Des weiteren können Riechstoffe oder Parfümöle, die das erfindungsgemäß einzusetzende Depotpräparat enthalten, an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulosebasierende Stoffe sein.
Riechstoffe oder Parfümöle, die das erfindungsgemäß einzusetzende Depotpräparat enthalten, können auch mikroverkapselt, sprühgetrocknet, als Einschluss-Komplex oder als Extrusions-Produkt vorliegen und in dieser Form dem zu parfümierenden Produkt hinzugefügt werden.
Gegebenenfalls können die Eigenschaften der derart modifizierten Riechstoffe oder Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.
Die Mikroverkapselung der Riechstoffe oder Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, gegebenenfalls in einem geeigneten Lösungsmittel, z.B. Isopropanol, erfolgen.
In Parfümkompositionen beträgt die eingesetzte Menge des erfindungsgemäß einzusetzenden Depotpräparates 0,01 bis 75 Gew.%, vorzugsweise 0,05 bis 50 Gew.-%, besonders bevorzugt ist eine Einsatzmenge von 0,5 bis 20 Gew.-%, bezogen auf das gesamte Parfümöl.
Riechstoffe oder Parfümöle, die das erfindungsgemäß einzusetzende Depotpräparat enthalten, können in konzentrierter Form, in Lösungen oder in oben beschriebener modifizierter
Form für die Herstellung von z.B. kosmetischen Pflege-Produkten verwendet werden. Hierbei insbesondere für Waschprodukte oder andere Produktapplikationen wo ein langanhaltender Geruchseindruck auf der Haut oder dem Haar erwünscht ist. Als Beispiele seien hier genannt: Parfüm-Extraits, Eau de Parfum, Eau de Toilettes, Rasierwässer, Eau de Colognes, Pre-shave-Produkte, Splash-Colognes, Körperpflegemittel wie z.B. feste und flüssige Seife, Duschgele, Shampoos, Rasierseifen, Rasierschäume, Badeöle, kosmetische Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargelen, festigende Haarlotionen, Haarspülungem permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkte der dekorativen Kosmetik wie z.B. Lidschatten, Nagellacke, Make-ups, Lippenstifte, Mascara.

Im Sinne der Erfindung werden Riechstoffe oder Parfümöle, die das erfindungsgemäß einzusetzende Depotpräparat enthalten, in konzentrierter Form, in Lösungen oder in oben beschriebener modifizierter Form für die Herstellung von Haarpflegeprodukten, Deodorantien und Antiperspirantien und hierbei insbesondere für die Herstellung von permanenten Haarfärbemitteln eingesetzt werden.

Weiterhin können Riechstoffe oder Parfümöle, die das erfindungsgemäß einzusetzende Depotpräparat enthalten, in konzentrierter Form, in Lösungen oder in oben beschriebener modifizierter Form für die Herstellung von z.B. Haushaltprodukten, wie Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern,

Wäscheweichspülern, Waschseifen, Waschtabletten, Desinfektionsmitteln, Oberflächendesinfektionsmitteln eingesetzt werden.

Bevorzugt können Riechstoffe oder Parfümöle, die das erfindungsgemäß einzusetzende Depotpräparat enthalten, in konzentrierter Form, in Lösungen oder in oben beschriebener modifizierter Form für die Herstellung von flüssigen oder pulverförmigen Waschmitteln eingesetzt werden.

Darüber hinaus kann das erfindungsgemäß einzusetzende Depotpräparat zur Aromatisierung von beispielsweise Verpackungsprodukten oder Nahrungsmitteln sowie deren Anwendungsformen für die Verwendung als Lebensmittel zum menschlichen oder tierischen Verzehr eingesetzt werden.

Im besonderen bevorzugte zu aromatisierende Produkte sind z.B. Süßwaren, Backwaren, Schokoladen, Gelatinewaren, Bonbons, alkoholische Getränke, nicht alkoholische Getränke, Eiscreme, Joghurt, Milchgetränke, Suppen, Soßen, Snacks, Kaugummi, Mundwasser, Fleisch- und Wurstwaren, Pflanzenproteinwaren, Fisch, Käse und Babynahrung.

Die erfindungsgemäß einzusetzenden Acylale der Formel (I), worin $R^2 = R^3$ ist und die vorgenannte Bedeutung haben, können nach dem Fachmann wohlvertrauten Methoden hergestellt werden (T.W. Greene, P.G.M. Wuts, Protective Groups in Organic Synthesis, 2nd Edition, John Wiley & Sons, Inc., New York 1991). Typischerweise setzt man den Aldehyd mit einem Carbonsäureanhydrid in Gegenwart von katalytischen Mengen Säure um. Säuren, die man für die Umsetzung benutzen kann sind: Schwefelsäure, Eisenchlorid, Phosphortrichlorid und saurer Ionentauscher. Weitere Verbindungen, die die Acylalbildung katalysieren sind: N-Bromsuccinimid (Karimi, B.; Seradj, H.; Ebrahimian, G.R., Synlett, 623-624, 2000), Kupfertriflat (Chandra, K.L.; Saravanan, P.; Singh, V.K., Synlett, 359-360, 2000), Bortrifluorid (Sydnes, L.K.; Sandberg, M., Tetrahedron, 53, 12679-12690, 1997), LiBr (Kumar, H.M.S.; Reddy, B.V.S.; Reddy, P.T.; Yadav, J.S., J. Chem. Res. (S), 86-87, 2000) $TiO_2/SO_4^{2-}$

Supersäure (Jin, T.-S.; Ma, Y.-R.; Sun, X.; Liang, D.; Li, T.-S., J. Chem. Res. (S), 96-97, 2000). Bevorzugte Säuren sind Eisenchlorid und Schwefelsäure.

Für die Herstellung der erfindungsgemäß einzusetzenden Acylale der allgemeinen Formel (I), worin $R^2 \neq R^3$ ist und auch die vorgenannte Bedeutung haben, setzt man den Aldehyd mit einem 1:1-Gemisch zweier Carbonsäureanhydride in Gegenwart katalytischer Mengen Säure um. Säuren die man für die Umsetzung benutzen kann sind im Absatz zuvor beschrieben. Das erhaltene Reaktionsgemisch kann sehr einfach durch Destillation oder Chromatographie getrennt werden.

Die folgenden, nicht limitierenden Beispiele erläutern die Erfindung.

**Allgemeine Vorschrift zur Herstellung der symmetrischen Acylale ($R^2 = R^3$)**

Man legt 15 mmol $FeCl_3$ in 1.0 mol Carbonsäureanhydrid vor und tropft anschließend 0.5 mol Aldehyd, gelöst in 0.5 mol Carbonsäureanhydrid so zu, dass die Temperatur 30°C nicht übersteigt. Nach beendeter Reaktion gibt man die Reaktionslösung auf 360 ml Cyclohexan/Wasser (5:1), trennt die Phasen und wäscht die organische Phase bis zur Neutralität mit gesättigter $NaHCO_3$-Lösung. Nachfolgend wird die organische Phase mit $Na_2SO_4$ getrocknet, abfiltriert und von Lösungsmittel befreit. Die Reinigung des Rohproduktes erfolgt über Destillation.

**Beispiel 1:**

(2,4-Dimethyl-3-cyclohexen-1-yl)(propionyloxy)methylpropionat

$^{1}$H-NMR (200 MHz, $CDCl_3$): δ (ppm) = 0.89 (d, J = 7.0 Hz, 3H), 1.14 (t, J = 7.5 Hz, 6H), 1.47-1.55 (m, 2H), 1.63-1.67 (m, 3H), 1.86-2.07 (m, 4H), 2.35 (q, J = 7.5 Hz, 4H), 5.31-5.37 (m, 1H), 6.8 (d, J = 8.1 Hz, 1H).
$^{13}$C-NMR (50 MHz, $CDCl_3$): δ (ppm) = 8.9, 15.6, 18.6, 20.7, 23.3, 27.4, 27.5, 29.7, 30.1, 40.7, 91.3, 126.8, 132.8, 172.4, 172.5.

**Beispiel 2:**

(Butyryloxy)(2,4-dimethyl-3-cyclohexen-1-yl)methylbutyrat

$^{1}$H-NMR (200 MHz, $CDCl_3$): δ (ppm) = 0.89 (d, J = 7.0 Hz, 3H), 0.95 (t, J = 7.5 Hz, 6H), 1.47-1.55 (m, 2H), 1.62-1.71 (m, 7H), 1.86-2.06 (m, 4H), 2.28-2.33 (m, 4H), 5.31-5.37 (m, 1H), 6.8 (d, J = 8.1 Hz, 1H).
$^{13}$C-NMR (50 MHz, $CDCl_3$): δ (ppm) = 13.5 (2C), 13.6, 15.6, 18.2, 18.6, 23.3, 29.7, 30.1, 36.0, 36.1, 40.6, 91.2, 126.7, 132.8, 171.6, 171.7.

**Beispiel 3:**

(Butyryloxy)[4-(4-methyl-3-pentenyl)-3-cyclohexen-1-yl]methylbutyrat

$^{1}$H-NMR (200 MHz, $CDCl_3$): δ (ppm) = 0.95 (t, J = 7.3 Hz, 6H), 1.60 (s, 3H), 1.61-1.68 (m, 4H), 1.69 (s, 3H), 1.76-1.84 (m, 1H), 1.88-2.10 (m, 10H) 2.28-2.34 (m, 4H), 5.09 (tq, J = 6.8, 1.3 Hz, 1H), 5.35-5.43 (m, 1H), 6.75 (d, J = 5.4 Hz, 1H).
$^{13}$C-NMR (50 MHz, $CDCl_3$): δ (ppm) = 13.6 (2C), 17.6, 18.3 (2C), 23.4, 25.6, 25.7, 26.4, 27.8, 36.0 (2C), 37.3, 37.6, 91.9, 118.8, 124.2, 131.4, 137.7, 171.7, 171.8.

**Beispiel 4:**

[4-(4-Methyl-3-pentenyl)-3-cyclohexen-1-yl] (propionyloxy)methylpropionat

$^{1}$H-NMR (200 MHz, $CDCl_3$): δ (ppm) = 1.15 (t, J = 7.5 Hz, 6H), 1.60 (s, 3H), 1.68 (s, 3H), 1.75-1.84 (m, 1H), 1.89-2.10 (m, 10H), 2.30-2.42 (m, 4H), 5.09 (tq, J = 6.8, 1.3 Hz, 1H), 5.35-5.43 (m, 1H), 6.75 (d, J = 5.4 Hz, 1H).
$^{13}$C-NMR (50 MHz, $CDCl_3$): δ (ppm) = 8.8 (2C), 23.3, 25.6, 25.7 (2C), 26.4, 27.4 (2C), 27.8, 37.3, 37.5, 91.9, 118.8, 124.2, 131.5, 137.7, 172.6, 172.7.

**Beispiel 5:**

(Butyryloxy)(phenyl)methylbutyrat

$^{1}$H-NMR (200 MHz, $CDCl_3$): δ (ppm) = 0.95 (t, J = 7.4 Hz, 6H), 1.67 (sex, J = 7.4 Hz, 4H), 2.32-2.40 (m, 4H), 7.37-7.44 (m, 3H), 7.48-7.55 (m, 2H), 7.71 (s, 1H).
$^{13}$C-NMR (50 MHz, $CDCl_3$): δ (ppm) = 13.5 (2C), 18.2 (2C), 35.9 (2C), 89.4, 126.5 (2C), 128.5 (2C), 129.5, 135.7, 171.3 (2C).

**Beispiel 6:**

3-(4-tert-Butylphenyl)-1-(butyryloxy)propylbutyrat

$^{1}$H-NMR (200 MHz, $CDCl_3$): δ (ppm) = 0.95 (t, J = 7.2 Hz, 6H), 1.30 (s, 9H), 1.65 (sex, J = 7.2 Hz, 4H), 2.04-2.16 (m, 2H), 2.24-2.33 (m, 4H), 2.63-2.74 (m, 2H), 6.87 (t, J = 5.5 Hz, 1H), 7.12 (d, J = 8.0 Hz, 2H), 7.31 (d, J = 8.0 Hz, 2H).
$^{13}$C-NMR (50 MHz, $CDCl_3$): δ (ppm) = 13.5 (2C), 18.1 (2C), 29.1, 31.3 (3C), 34.3, 34.6, 35.8 (2C), 89.8, 125.2 (2C), 127.8 (2C), 137.3, 148.7, 171.4 (2C).

**Beispiel 7:**

1-(Heptanoyloxy)decylheptanoat

$^{1}H$-NMR (200 MHz, $CDCl_3$): δ (ppm) = 0.83-0.93 (m, 9H), 1.22-1.38 (m, 28H), 1.53-1.77 (m, 4H), 2.31, (t, J = 7.4 Hz, 4H), 6.79 (t, J = 5.6 Hz, 1H).
$^{13}C$-NMR (50 MHz, $CDCl_3$): δ (ppm) = 14.0 (2C), 14.1, 22.4 (2C), 22.6, 23.4, 24.6 (2C), 28.6 (2C), 29.1, 29.2, 29.4 (2C), 31.4 (2C), 31.8, 33.2, 34.1 (2C), 90.2, 171.7 (2C).

**Beispiel 8:**

1-(Acetyloxy)heptylacetat

$^{1}H$-NMR (200 MHz, $CDCl_3$): δ (ppm) = 0.88 (t, J = 6.5 Hz, 3H), 1.23-1.42 (m, 8H), 1.70-1.82 (m, 2H), 2.08 (s, 6H), 6.77 (t, J = 5.6 Hz, 1H).
$^{13}C$-NMR (50 MHz, $CDCl_3$): δ (ppm) = 14.0, 20.8 (2C), 22.5, 23.3, 28.8, 31.6, 33.1, 90.4, 168.9 (2C).

**Beispiel 9:**

(Acetyloxy)(2,4-dimethyl-3-cyclohexen-1-yl)methylacetat

Angabe der Isomerie: cis/trans = 2.3:1.
$^{1}H$-NMR (200 MHz, $CDCl_3$): δ (ppm) = 0.88 (d, J = 7.0 Hz, 3H), 1.43-1.57 (m, 2H), 1.62-1.67 (m, 3H), 1.90-2.04 (m, 3H), 2.08 (s, 6H), 2.26-2.40 (m, 1H), 5.30-5.37 (m, 1H), 6.77 (d, J = 8.2 Hz, 1H).
$^{13}C$-NMR (50 MHz, $CDCl_3$): δ (ppm) = 15.5, 18.5, 20.8 (2C), 23.3, 29.6, 30.1, 40.5, 91.3, 126.5, 132.6, 168.9 (2C).

**Beispiel 10:**

1-(Acetyloxy)-2-methylundecylacetat

$^{1}H$-NMR (200 MHz, $CDCl_3$): δ (ppm) = 0.88 (t, J = 6.3 Hz, 3H), 0.95 (d, J = 6.7 Hz, 3H), 1.20-1.32 (m, 16H), 1.77-1.93 (m, 1H), 2.08 (s, 6H), 6.69 (d, J = 4.4 Hz, 1H).
$^{13}C$-NMR (50 MHz, $CDCl_3$): δ (ppm) = 13.3, 14.1, 20.7, 20.8, 22.7, 26.8, 29.3, 29.5, 29.6, 29.7, 30.8, 31.9, 36.3, 92.3, 168.9 (2C).

**Allgemeine Vorschrift zur Herstellung** der **unsymmetrischen Acylale** ($R^2 \neq R^3$)

Man legt 15 mmol $FeCl_3$ in 1.0 mol eines 1:1-Gemisches von zwei Carbonsäureanhydriden vor und tropft anschließend 0.5 mol Aldehyd, gelöst in 0.5 mol eines 1:1-Gemisches von zwei Carbonsäureanhydriden so zu, dass die Temperatur 30°C nicht übersteigt. Nach beendeter Reaktion gibt man die Reaktionslösung auf 360 ml Cyclohexan/Wasser (5:1), trennt die Phasen und wäscht die organische Phase bis zur Neutralität mit gesättigter $NaHCO_3$-Lösung. Nachfolgend wird die organische Phase mit $Na_2SO_4$ getrocknet, abfiltriert und von Lösungsmittel befreit. Die Reinigung des Produktgemisches erfolgt über Destillation oder Chromatographie.

**Beispiel 11:**

1-(Acetyloxy)-3-(4-tert-butylphenyl)propylhexanoat

$^{1}H$-NMR (200 MHz, $CDCl_3$): δ (ppm) = 0.90 (t, J = 7.3 Hz, 3H), 1.30 (s, 9H), 1.24-1.39 (m, 4H), 1.51-1.75 (m, 2H), 2.05 (s, 3H), 2.04-2.16 (m, 2H), 2.26-2.37 (m, 2H), 2.63-2.74 (m, 2H), 6.85 (t, J = 5.4 Hz, 1H), 7.12 (d, J = 8.1 Hz, 2H), 7.31 (d, J = 8.1 Hz, 2H).
$^{13}C$-NMR (50 MHz, $CDCl_3$): δ (ppm) = 13.9, 20.7, 22.3, 24.3, 29.0, 31.1, 31.3 (3C), 33.9, 34.3, 34.6, 89.9, 125.2 (2C), 127.8 (2C), 137.3, 148.7, 168.8, 171.6.

**Beispiel 12:**

1-(Acetyloxy)-3-(4-tert-butylphenyl)propylheptanoat

$^1$H-NMR (200 MHz, $CDCl_3$): δ (ppm) = 0.88 (t, J = 7.1 Hz, 3H), 1.30 (s, 9H), 1.24-1.38 (m, 6H), 1.52-1.71 (m, 2H), 2.05 (s, 3H), 2.03-2.18 (m, 2H), 2.26-2.38 (m, 2H), 2.63-2.74 (m, 2H), 6.85 (t, J = 5,4 Hz, 1H), 7.12 (d, J = 8.0 Hz, 2H), 7.31 (d, J = 8.0 Hz, 2H).
$^{13}$C-NMR (50 MHz, $CDCl_3$): δ (ppm) = 14.0, 20.7, 22.4, 24.5, 28.6, 29.0, 31.3 (3C), 31.4, 33.9, 34.3, 34.6, 89.9, 125.2 (2C), 127.8 (2C), 137.3, 148.7, 168.7, 171.6.

**Beispiel 13:**

1-(Acetyloxy)decylhexanoat

$^1$H-NMR (200 MHz, $CDCl_3$): δ (ppm) = 0.84-0.96 (m, 6H), 1.22-1.40 (m, 18H), 1.55-1.82 (m, 4H), 2.07 (s, 3H), 2.44 (t, J = 7.4 Hz, 2H), 6.78 (t, J = 5.6 Hz, 1H).
$^{13}$C-NMR (50 MHz, $CDCl_3$): δ (ppm) = 13.9, 14.1, 20.8, 22.3, 22.7, 23.4, 24.3, 29.1, 29.3, 29.4 (2C), 31.1, 31.9, 33.2, 34.0, 90.4, 168.9, 171.7.

**Beispiel 14**

1-(Acetyloxy)decylheptanoat

$^1$H-NMR (200 MHz, $CDCl_3$): δ (ppm) = 0.83-0.95 (m, 6H), 1.22-1.40 (m, 20H), 1.54-1.82 (m, 4H), 2.07 (s, 3H), 2.45 (t, J = 7.4 Hz, 2H), 6.78 (t, J = 5.6 Hz, 1H).
$^{13}$C-NMR (50 MHz, $CDCl_3$): δ (ppm) = 14.0, 14.1, 20.8, 22.5, 22.7, 23.4, 24.6, 28.6, 29.1, 29.3, 29.5 (2C), 31.4, 31.9, 33.2, 34.1, 90.4, 168.9, 171.7.

Die erfindungsgemäß einzusetzenden Depotpräparate wurden in zahlreiche Verbraucherprodukte eingearbeitet und deren anwendungstechnischen Eigenschaften mit verschiedenen Methoden untersucht. Bei der Herstellung der Formulierungen für die Verbraucherprodukte wurden molare Äquivalente der Aldehyde in Form der Acylale bzw. in Form der freien Aldehyde eingesetzt, um eine Vergleichbarkeit zu gewährleisten.

Methode 1: Lagerstabilität

Die Lagerstabilität eines Riechstoffes bzw. eines Depotpräparates wird durch die prozentuale Menge des nach Lagerung noch vorhandenen Stoffes definiert.

$$\frac{\text{Menge nach der Lagerung}}{\text{Menge vor der Lagerung}} * 100\% = \text{Lagerstabilität [\%]}$$

Zur Bestimmung und zum Vergleich der Lagerstabilität werden sowohl das Depotpräparat (DP) bestehend aus einem oder mehreren Acylalen und die korrespondierenden Aldehyde (AL) in separate Muster ($M_{DP}$ und $M_{AL}$) der gleichen Formulierung eines Verbraucherproduktes wie z.B. Waschmittel, Shampoo oder Seife eingearbeitet. Anschließend werden die separaten Muster in Portionen geteilt. Die eine Portion der Muster $M_{DP}$ und $M_{AL}$ werden unverzüglich einer geeigneten Extraktion und einer analytischen Messung unterzogen, um die Menge an Depotpräparat bzw. Aldehyd vor der Lagerung zu bestimmen. Bei der analytischen Untersuchung durch z.B.

Gaschromatographie wird zur Quantifizierung ein geeigneter Standard verwendet. Die zweite Portion wird einer Lagerung bei erhöhter Temperatur für eine definierte Zeit unterzogen und anschließend mit den gleichen Methoden extrahiert und quantifiziert.

Methode 2: Geruchliche Bewertung

Die geruchliche Bewertung eines Depotpräparates an sich bzw. im Vergleich mit den korrespondierenden Aldehyden wird durch ein Gruppe geschulter Personen durchgeführt. Hierbei werden die geruchliche Stärke des Verbraucherproduktes bzgl. des verwendeten Aldehydes in der Anwendung beurteilt. Das Verbraucherprodukt wird gemäß

seiner Bestimmung zum Auftragen auf die Haut oder zum Waschen von Wäsche, Haut oder Haar verwendet. Während der Anwendung wird dann z.B. das Verbraucherprodukt selbst, dessen wässrige Lösungen, die feuchte oder trockene Wäsche bzw. die feuchte oder trockene Haut geruchlich auf einer Skala von 1 (schwacher Geruch) bis 6 (starker Geruch) beurteilt.

Methode 3: Freisetzungsrate im Gasraum

Die analytische Messung der Konzentration von Riechstoffen selbst, den Depotpräparaten und den Riechstoffen freigesetzt aus den Depotpräparaten wird mittels der Gaschromatographie durchgeführt. Hierbei können weiterhin verschiedene Injektionsmethoden, wie z.B. die Thermodesorption, die Flüssiginjektion und die Gasinjektion verwendet werden.

Vor der analytischen Messung von Riechstoffen können verschiedene Anreicherungsverfahren wie z.B. die Extraktion, die Aufkonzentration oder die Adsorption verwendet werden. Als Extraktionsmittel für flüssig-flüssig oder flüssig-fest Extraktionen sind z.B. Lösungsmittel wie z.B. Kohlendioxid, Ether, Ketone, Kohlenwasserstoffe, Alkohole, Wasser und Ester geeignet.

Weiterhin kann durch Ausfrieren eines statischen oder dynamischen Gasraumes über dem parfümierten Produkt bzw. mit dem parfümierten Produkt behandelte Substrate wie Haar, Textilien oder Haut mittels Kühlfallen eine Anreicherung bzw. Aufkonzentrierung erreicht werden.

Für die Adsorption bzw. Extraktion von Riechstoffen aus einem statischen oder dynamischen Gasraum sind oberflächenaktive Adsorptionsmittel wie z.B. Haar, Textilien, Keramik, Kunststoffe, Aktivkohle sowie Poly-2,6-diphenyl-p-phenylenoxid (Tenax®) und quervernetzte poröse Polymere auf Basis von Styrol, Ethylvinylbenzol, Vinylpyrrolidon, Vinylpyridin und Ethylenglykol-dimethacrylat (Poropax®-Serie) geeignet. Die an diesen Adsorptionsmitteln angereicherten Riechstoffe werden anschließend durch Wärme (Thermodesorption) oder Lösungsmittel desorbiert und können dann analysiert werden.

**Beispiel 15: Pulverwaschmittel (Vergleichsbeispiel)**

Die Waschmittel A und B wurden sowohl direkt als auch nach Lagerung für vier Wochen zum Waschen verwendet bzw. analytisch untersucht.

Tabelle 1: Pulverwaschmittelformulierung

| Inhaltsstoffe | | A | B |
|---|---|---|---|
| Pulverwaschmittel | | 99,7 | 99,84 |
| DP Beispiel 3 (4) | (Butyryloxy)[4-(4-methyl-3-pentenyl)-3-cyclohexen-1-yl]methylbutyrat | 0,3 | |
| Vertomugal (4) | 1-Formyl-4-(4-methyl-3-pentenyl)-3-cyclohexen | | 0,16 |

Geruchliche Beurteilung:

Baumwollgewebe und synthetisches Gewebe wurden zusammen in zwei verschiedenen Maschinen zeitgleich mit den oben genannten Waschmitteln A und B gewaschen und im feuchten und im trockenen Zustand geruchlich bewertet: Die geruchliche Stärke der Wäschestücke, die mit dem Waschmittel A mit Depotpräparat gewaschen wurden war deutlich höher als die geruchliche Stärke der Wäschestücke, die mit dem Waschmittel B mit dem freien Aldehyd gewaschen wurden.

Lagerstabilität:

Die Lagerstabilität des freien Aldehydes nach einem Monat betrug 34%. Die aus dem freien Aldehyd gebildete Säure erzeugte eine unangenehme geruchliche Note. Die Lagerstabilität des Aldehydes im Depotpräparat betrug 93% und war damit deutlich höher.

Freisetzungsrate:

Die gewaschenen Textilien A und B wurden jeweils in separate Glasflaschen überführe. Anschließend wurde mittels SPME-Analyse ("Solid Phase Microextraction") bzw. direkter Gasraumanalyse die relative Konzentration an freiem Aldehyd analysiert. In dem Gasraum über der mit dem Waschmittel A gewaschenen feuchten Wäsche wurden

über fünfmal mehr freier Aldehyd gefunden. In dem Gasraum über der mit dem Waschmittel A gewaschenen trockenen Wäsche wurden siebenmal mehr des Aldehydes gefunden.

**Beispiel 16: Seife (Vergleichsbeispiel)**

Die folgende Seifenformulierung kann nach allgemein bekannten Methoden hergestellt werden. Die Angaben beziehen sich auf Gewichtsprozente. Die erhaltenen Seifen A bis F wurden sowohl direkt als auch nach Lagerung für vier Wochen zum Waschen verwendet bzw. analytisch untersucht.

**Tabelle 2:** Seifenformulierung

| Inhaltsstoffe | | A | B | C | D | E | F |
|---|---|---|---|---|---|---|---|
| Seifenbase (1) | Sodium Tallowate | 75,0 | 75,0 | 75,0 | 75,0 | 75,0 | 75,0 |
| Seifenbase (I) | Sodium Cocoate | 10,0 | 10,0 | 10,0 | 10,0 | 10,0 | 10,0 |
| Wasser | Water | 13,0 | 13,0 | 13,0 | 13,0 | 13,0 | 13,0 |
| Bayertitan AZ (2) | Titanium Dioxide | 0,3 | 0,3 | 0,3 | 0,3 | 0,3 | 0,3 |
| Tinopal CBS-X (3) | Disodium Distyrylbiphenyl Disulfonate | 0,2 | 0,2 | 0,2 | 0,2 | 0,2 | 0,2 |
| DP Beispiel 7 (4) | 1-(Heptanoyloxy)decylheptanoat | 0,3 | | | | | |
| Aldehyd C10 (4) | Decanal | | 0,12 | | | | |
| DP Beispiel 5 (4) | (Butyryloxy)(phenyl)methyl-butyrat | | | 0,3 | | | |
| Benzaldehyd (4) | Benzaldehyd | | | | 0,12 | | |
| DP Beispiel 6 (4) | 3-(4-tert-Butylphenyl)-1-(butyryloxy) propylbutyrat | | | | | 0,3 | |
| Bourgeonal (5) | 4-tert.-Butyldihydro-zimtaldehyd | | | | | | 0,16 |
| Lieferanten:<br>(1) Enzian Seifenfabrik, 72555 Metzingen, Germany<br>(2) Bayer AG, Bayerwerk, D-51368 Leverkusen, Germany<br>(3) Ciba Spezialitätenchemie AG, 4000 Basel, Switzerland<br>(4) Haarmann & Reimer GmbH, D-37603 Holzminden, Germany<br>(5) Quest International, Ashford, England | | | | | | | |

Geruchliche und farbliche Beurteilung:

Die Seifenformulierungen A bis F wurden für ca. drei Monate bei Raumtemperatur gelagert.

Die Seifen A, C und E welche die Depotpräparate beinhalten zeigten farblich nicht oder nur geringfügig Veränderung während die Seifen D und F eine gelbliche bzw. graue Verfärbung hatten. Durch die Verwendung der Depotpräparate wird eine Farbstabilität erzielt.

Nach der Lagerung wurden jeweils 1g der Seifen in 100g handwarmen Wasser aufgelöst bzw. die Seifenstücke zum Waschen von Haut verwendet.

In allen Fällen war der Dufteindruck über den wässrigen Lösungen der Seifen A, C und E, welche die Depotpräparate beinhalten, deutlich stärker als der Dufteindruck der Seifen B, D und F, welche die freien Aldehyde beinhalten.

Der Dufteindruck von der gewaschenen Haut, welche mit den Seifen A, C und E gewaschen wurde, war ebenfalls höher und lang anhaltender als der Dufteindruck nach Waschen mit den Seifen B, D und F.

Lagerstabilität:

Die Seifenformulierungen A bis F wurden für ca. einen Monat bei Raumtemperatur in der Dunkelheit gelagert. Alle Depotpräparate zeigten eine deutlich höhere Lagerstabilität als die korrespondierenden Aldehyde.

**Tabelle 3:** Lagerstabilität von Depotpräparaten und freien Aldehyden in Seifen

| Inhaltsstoffe | | Lagerstabilität [%] |
|---|---|---|
| DP Beispiel 7 (4) | 1-(Heptanoyloxy)-decylheptanoat | 95 |
| Aldehyd C10 (4) | Decanal | 32 |
| DP Beispiel 5 (4) | (B utyryloxy)(phenyl)-methylbutyrat | 93 |
| Benzaldehyd (4) | Benzaldehyd | 45 |
| DP Beispiel 6 (4) | 3-(4-tert-Butylphenyl)-1-(butyryloxy)propylbutyrat | 93 |
| Bourgeonal (5) | 4-tert.-Butyldihydro-zimtaldehyd | 27 |

Freisetzungsrate:

Zur Bestimmung der Hydrolysegeschwindigkeit wurde das Depotpräparat zu einer 1%igen wässrigen Seifenlösung gegeben und die Konzentration des Depotpräparates bzw. des freien Aldehydes durch SPME-Analyse im Gasraum gemessen.

Die Depotpräparate in den Seifen A, C und E zeigten eine spontane Hydrolyse zu den korrespondierenden Aldehyden. Bereits nach 5 Minuten war das Depotpräparat vollständig hydrolysiert.

Durch geeignete Wahl der Reste im Depotpräparat kann auch eine verzögerte bzw. unvollständige Hydrolyse erzielt werden. Hierdurch kann dann im Waschprozess ein Teil des Depotpräparates auf die Haut übertragen werden und anschließend durch die langsamere Spaltung der Acylale ein dauerhafter Dufteindruck auf der Haut erzielt werden.

**Beispiel 17: Shampoo (Vergleichsbeispiel)**

Die folgende Shampooformulierung kann nach allgemein bekannten Methoden hergestellt werden. Die Angaben beziehen sich auf Gewichtsprozente.

**Tabelle** 4: Shampooformulierung

| Inhaltsstoffe | | A | B |
|---|---|---|---|
| Plantacare PS 10 (1) | Sodium Laureth Sulfate (and) Lauryl Glycoside | 20,000 | 20,000 |
| Entmineralisiertes Wasser | Water (Aqua) | bis 100% | bis 100% |
| Natriumchlorid | Sodium Chloride | 1,400 | 1,400 |
| Citronensäure 10,0 % Lösung | Citric Acid | 1,650 | 1,650 |
| Phenonip (2) | Phenoxyethanol (land) Methyl paraben (and) Ethylparaben (and) Propylparaben (and) Butylparaben | 0,500 | 0,500 |
| DP Beispiel 7 (4) | 1-(Heptanoyloxy)decylheptanoat | 0,3 | |
| Aldehyd C10 (4) | Decanal | | 0,12 |

Geruchliche Beurteilung:

Die erhaltenen Shampoos A und B wurden zum Waschen von Haarsträhnen bzw. in einem sogenannten Halbseitenwaschtest an Probanden verwendet. Bei dem Halbseitenwaschtest wurde den Probanden jeweils eine Hälfte der Haare mit Shampoo A und die andere Hälfte der Haare mit Shampoo B gewaschen.

Die mit Shampoo B gewaschenen Haarsträhnen hatten direkt nach der Wäsche eine geringfügig höhere geruchliche Stärke im Vergleich zu den Haarsträhnen welche mit dem Shampoo A gewaschen wurden. Allerdings nahm die geruchliche Intensität der mit Shampoo B gewaschenen Haare rasch ab und war nach ca. vier Stunden nicht mehr von einer neutralen Probe unterscheidbar.

Die gleiche Duftbewertung ergab sich auch für den Halbseitenwaschtest an Probanden. Allerdings wurde von der Hälfte, die mit dem Shampoo A gewaschen wurde ein lang andauernder Aldehydgeruch wahrgenommen. Auch noch nach mehreren Tagen war ein deutlicher Geruch des Aldehydes auf der Haarhälfte A wahrnehmbar. Durch die höhere Übertragung und anschließende langsame Spaltung des Depotpräparates zum freien Aldehyd kann ein lang anhaltender Dufteindruck erzielt werden. Hierdurch zeigt sich der Vorteil des Depotpräparates gegenüber dem freien

Aldehyd in der Anwendung.

**Beispiel 18: Permanentes Haarfärbemittel**

Stabilität in der Entwicklermasse:

Die Formulierung der Entwicklermasse enthält typischerweise Wasser, Wasserstoffperoxid, Säuren wie z.B. Phosphorsäure, Citronensäure usw., Verdicker, Emulgatoren, Konservierungsmittel, Lösungsmittel und weitere Hilfsstoffe. Zu der Formulierung der Entwicklermasse wird 1% DP Beispiel 5 gegeben.

Die Entwicklerformulierung, die 1% DP Beispiel 5 enthält, wird für eineinhalb Monate bei Raumtemperatur gelagert.

**Tabelle 5:** Lagerstabilität von Depotpräparat in Entwicklermasse

| | **Stabilität [%] 0 Tage** | **Stabilität [%] 13 Tage** | **Stabilität [%] 28 Tage** | **Stabilität [%] 42 Tage** |
|---|---|---|---|---|
| DP Beispiel 5 (4) | 100 | 100 | 98 | 100 |

Das Depotpräparat ist über den eineinhalbmonatigen Zeitraum farblich, geruchlich und analytisch stabil.

Freisetzungsrate während der Haarfärbung:

Zur Bestimmung der Hydrolysegeschwindigkeit wurden die drei Depotpräparate DP Beispiel 8, DP Beispiel 9 und DP Beispiel 10 in einer Konzentration von jeweils 0,3 % zu der Entwicklermasse gegeben. Anschließend fügt man zu der Entwicklermasse im Verhältnis 1:1 die ammoniakalische Färbelösung, welche sich aus 2 bis 16 % Ammoniak und/oder Ersatzstoffen wie z.B. Alkanolamine, insbesondere Monoethanolamin, Wasser, Verdicker, Emulgator, Konsistenzbildner, Reaktivfarbstoffe, Lösungsmittel, Komplexbildner, Stabilisatoren und Konservierungsmittel zusammensetzt, hinzu. Jetzt werden in definierten zeitlichen Abständen Proben aus der Haarfärbelösung entnommen, die Proben werden neutralisiert, mit Lösungsmittel extrahiert und der Gehalt an Depotpräparat und freigesetztem Aldehyd mittels Gaschromatographie unter Verwendung eines internen und externen Standards bestimmt.

**Tabelle 6:** Freisetzungsrate während der Haarfärbung

| **Zeit (min)** | **relativer GC Gehalt [%]** | | | | | |
|---|---|---|---|---|---|---|
| | **Aldehyd C7** | **Vertocitral** | **2-Methyl-undecanal** | **DP Beispiel 8** | **DP Beispiel 9** | **DP Beispiel 10** |
| 1 | 1,4 | 2,1 | 2,8 | 22,9 | 28,9 | 48,2 |
| 5 | 17,1 | 33,6 | 32,8 | 0,0 | 2,5 | 5,1 |
| 10 | 19,3 | 33,6 | 47,1 | 0,0 | 0,5 | 2,0 |
| 20 | 12,2 | 19,3 | 20,7 | 0,0 | 0,0 | 0,0 |
| 30 | 11,4 | 15,7 | 16,4 | 0,0 | 0,0 | 0,0 |

Die Depotpräparate in der Formulierung für Haarfärbung zeigten nach Vereinigung der Entwicklermasse mit der ammoniakalischen Haarfärbelösung eine spontane Hydrolyse zu den korrespondierenden Aldehyden. Bereits nach 5 Minuten waren die Depotpräparate nahezu vollständig hydrolysiert.

Geruchliche Beurteilung:

**Tabelle 7:** Entwicklerformulierung

| **Inhaltsstoffe** | **A** | **B** | **C** | **D** |
|---|---|---|---|---|
| Entwicklermasse | 100% | 99,7% | 99,7 % | 99,7 % |

(fortgesetzt)

| Inhaltsstoffe | A | B | C | D |
|---|---|---|---|---|
| DP Beispiel 8 (1-(Acetyloxy)heptylacetat) | | 0,3% | | |
| DP Beispiel 9 (Acetyloxy)(2,4-dimethyl-3-cyclo-hexen-1-yl)methylacetat) | | | 0,3 % | |
| DP Beispiel 10 (1-(Acetyloxy)-2-methylundecylacetat | | | | 0,3% |

Die erhaltenen Entwickler A bis D wurden im Verhältnis 1:1 mit der ammoniakalischen Färbelösung vereinigt, zwei Minuten gerührt und anschließend auf vier Haarsträhnen aufgetragen. Unmittelbar nach dem Auftragen war bei den Haarsträhnen, die mit den Entwicklerformulierungen B bis D bestrichen wurden, ein deutlicher Aldehydgeruch wahrnehmbar. Der Geruchseindruck verstärkte sich bzw. blieb konstant über den Zeitraum von 30 Minuten.

**Tabelle 8:** Sensorische Aldehydintensität während des Haarfärbeprozesses

| Zeit (min) | Sensorische Intensität | | |
|---|---|---|---|
| | Aldehyd C7 | Vertocitral | 2-Methyl-undecanal |
| 1 | 4,0 | 4,6 | 4,6 |
| 2 | 5,0 | 5,4 | 5,4 |
| 4 | 6,0 | 5,8 | 5,8 |
| 8 | 6,2 | 6,0 | 6,0 |
| 15 | 6,2 | 6,0 | 6,0 |
| 25 | 4,4 | 5,4 | 5,4 |

Die Skala der sensorischen Intensität reicht von 1,0 = geruchslos bis zu 9,0 = sehr stark.

Hieraus ergibt sich überraschenderweise ein erheblicher Vorteil in der Verwendung der erfindungsgemäß einzusetzenden Depotpräparate zur gezielten Freisetzung von Aldehyden in Parfümölen für alkalische Haarfärbemittel.

**Patentansprüche**

1. Permanentes Haarfärbemittel, enthaltend Riechstoffe oder Parfümöl, wobei die Riechstoffe oder das Parfümöl ein Depotpräparat der Formel (I) enthalten,

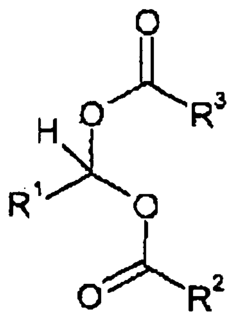

(I)

worin $R^1$, $R^2$ und $R^3$ - unabhängig voneinander - einen organischen Rest mit 1 bis 30 Kohlenstoffatomen bedeuten und die Verbindung der Formel (I) nach Hydrolyse oder enzymatischer Spaltung einen Aldehyd und zwei Carbonsäuren freisetzt.

2. Permanentes Haarfärbemittel nach Anspruch 1, enthaltend eine Entwicklermasse, wobei die Entwicklermasse das Depotpräparat der Formel (I) enthält.

3. Permanentes Haarfärbemittel nach Anspruch 1 oder 2, wobei die Riechstoffe oder das Parfümöl Extrakte aus natürlichen Rohstoffen, Kohlenwasserstoffe, aliphatische Alkohole, aliphatische Ketone, aliphatische Nitrile, aliphatische Carbonsäuren, acyclische Terpenalkohole, acyclische Terpenaldehyde, cyclische Terpenaldehyde, cyclische Alkohole, cycloaliphatische Alkohole, cyclische oder cycloaliphatische Ether, cyclische Ketone, cycloaliphatische Aldehyde oder Ketone, Ester cyclischer Alkohole oder Carbonsäuren, aromatische Kohlenwasserstoffe, araliphatische Alkohole, Ester araliphatischer Alkohole mit aliphatischen Carbonsäuren, aromatische oder araliphatische Aldehyde, Ketone oder Carbonsäuren, stickstoffhaltige, aromatische Verbindungen, Phenole, Phenylether oder Phenylester, heterocyclische Verbindungen oder Lactone enthalten.

4. Permanentes Haarfärbemittel nach einem der vorangehenden Ansprüche, wobei der Riechstoff oder das Parfümöl an einen Trägerstoff adsorbiert ist.

5. Permanentes Haarfärbemittel nach einem der Ansprüche 1 bis 3, wobei der Riechstoff oder das Parfümöl mikroverkapselt oder sprühgetrocknet ist oder als Einschluss-Komplex oder als Extrusions-Produkt vorliegt.

6. Verwendung von Riechstoffen oder Parfümölen für die Herstellung von permanenten Haarfärbemitteln, wobei die Riechstoffe oder Parfümöle ein Depotpräparat der Formel (I)

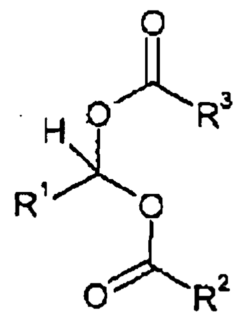

(I)

enthalten, worin $R^1$, $R^2$ und $R^3$ - unabhängig voneinander - einen organischen Rest mit 1 bis 30 Kohlenstoffatomen bedeuten und die Verbindung der Formel (I) nach Hydrolyse oder enzymatischer Spaltung einen Aldehyd und zwei Carbonsäuren freisetzt.

7. Verwendung nach Anspruch 6, wobei die Riechstoffe oder das Parfümöl Extrakte aus natürlichen Rohstoffen, Kohlenwasserstoffe, aliphatische Alkohole, aliphatische Ketone, aliphatische Nitrile, aliphatische Carbonsäuren, acyclische Terpenalkohole, acyclische Terpenaldehyde, cyclische Terpenaldehyde, cyclische Alkohole, cycloaliphatische Alkohole, cyclische oder cycloaliphatische Ether, cyclische Ketone, cycloaliphatische Aldehyde oder Ketone, Ester cyclischer Alkohole oder Carbonsäuren, aromatische Kohlenwasserstoffe, araliphatische Alkohole, Ester araliphatischer Alkohole mit aliphatischen Carbonsäuren, aromatische oder araliphatische Aldehyde, Ketone oder Carbonsäuren, stickstoffhaltige, aromatische Verbindungen, Phenole, Phenylether oder Phenylester, heterocyclische Verbindungen oder Lactone enthalten.

**Claims**

1. A permanent hair dye preparation containing odoriferous substances or perfume oil, the odoriferous substances or the perfume oil containing a depot preparation of the formula (I),

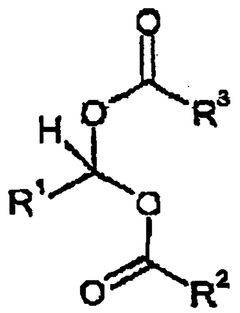

(I)

in which $R^1$, $R^2$ and $R^3$, mutually independently, mean an organic residue with 1 to 30 carbon atoms and, after hydrolysis or enzymatic cleavage, the compound of the formula (I) releases an aldehyde and two carboxylic acids.

2. A permanent hair dye preparation according to claim 1, containing a developer composition, the developer composition containing the depot preparation of the formula (I).

3. A permanent hair dye preparation according to claim 1 or claim 2, the odoriferous substances or the perfume oil containing extracts from natural raw materials, hydrocarbons, aliphatic alcohols, aliphatic ketones, aliphatic nitriles, aliphatic carboxylic acids, acyclic terpene alcohols, acyclic terpene aldehydes, cyclic terpene aldehydes, cyclic alcohols, cycloaliphatic alcohols, cyclic or cycloaliphatic ethers, cyclic ketones, cycloaliphatic aldehydes or ketones, esters of cyclic alcohols or carboxylic acids, aromatic hydrocarbons, araliphatic alcohols, esters of araliphatic alcohols with aliphatic carboxylic acids, aromatic or araliphatic aldehydes, ketones or carboxylic acids, nitrogenous, aromatic compounds, phenols, phenyl ethers or phenyl esters, heterocyclic compounds or lactones.

4. A permanent hair dye preparation according to any one of the preceding claims, the odoriferous substance or the perfume oil being adsorbed on a carrier.

5. A permanent hair dye preparation according to any one of claims 1 to 3, the odoriferous substance or the perfume oil being microencapsulated or spray-dried or assuming the form of an inclusion complex or an extrusion product.

6. Use of odoriferous substances or perfume oils for producing permanent hair dye preparations, the odoriferous substances or perfume oils containing a depot preparation of the formula (I)

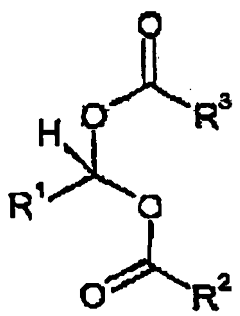

(I)

in which $R^1$, $R^2$ and $R^3$, mutually independently, mean an organic residue with 1 to 30 carbon atoms and, after hydrolysis or enzymatic cleavage, the compound of the formula (I) releases an aldehyde and two carboxylic acids.

7. Use according to claim 6, the odoriferous substances or the perfume oil containing extracts from natural raw materials, hydrocarbons, aliphatic alcohols, aliphatic ketones, aliphatic nitriles, aliphatic carboxylic acids, acyclic terpene alcohols, acyclic terpene aldehydes, cyclic terpene aldehydes, cyclic alcohols, cycloaliphatic alcohols, cyclic or cycloaliphatic ethers, cyclic ketones, cycloaliphatic aldehydes or ketones, esters of cyclic alcohols or carboxylic acids, aromatic hydrocarbons, araliphatic alcohols, esters of araliphatic alcohols with aliphatic carboxylic acids, aromatic or araliphatic aldehydes, ketones or carboxylic acids, nitrogenous, aromatic compounds, phenols, phenyl ethers or phenyl esters, heterocyclic compounds or lactones.

## Revendications

1. Teinture de cheveux permanente, contenant des substances odoriférantes ou de l'huile de parfum, dans laquelle les substances odoriférantes ou l'huile de parfum contiennent une préparation dépôt de la formule (1)

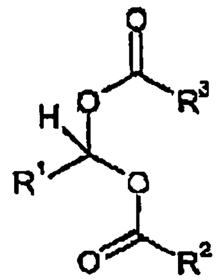

(I)

dans laquelle $R^1$, $R^2$ et $R^3$ signifient indépendamment l'un de l'autre un radical organique avec 1 à 30 atomes de carbone et dans laquelle, après une hydrolyse ou une élimination enzymatique, le composé de la formule (I) libère un aldéhyde et deux acides carboxyliques.

2. Teinture de cheveux permanente selon la revendication 1, contenant une masse de révélateur, la masse de révélateur contenant le produit dépôt de la formule (I).

3. Teinture de cheveux permanente selon la revendication 1 ou 2, dans laquelle les substances odoriférantes ou l'huile de parfum contiennent des extraits de matières premières naturelles, des hydrocarbures, des alcools aliphatiques, des cétones aliphatiques, des nitriles aliphatiques, des acides carboxyliques aliphatiques, des alcools terpéniques acycliques, des aldéhydes terpéniques acycliques, des aldéhydes terpéniques cycliques, des alcools cycliques, des alcools cycloaliphatiques, des éthers cycliques ou cycloaliphatiques, des cétones cycliques, des aldéhydes ou des cétones cycloaliphatiques, des esters d'alcools ou d'acides carboxyliques cycliques, des hydrocarbures aromatiques, des alcools araliphatiques, des esters d'alcools araliphatiques avec des acides carboxyliques aliphatiques, des aldéhydes, des cétones ou des acides carboxyliques aromatiques ou araliphatiques, des composés azotés aromatiques, des phénols, des phényléthers ou des phénylesters, des composés hétérocycliques ou des lactones.

4. Teinture de cheveux permanente selon l'une quelconque des revendications précédentes, dans laquelle la substance odoriférante ou l'huile de parfum est absorbée sur une substance porteuse.

5. Teinture de cheveux permanente selon l'une quelconque des revendications 1 à 3, dans laquelle la substance odoriférante ou l'huile de parfum est micro-encapsulée ou séchée par pulvérisation ou est présente comme complexe d'inclusion ou comme produit d'extrusion.

6. Utilisation de substances odoriférantes ou d'huiles de parfum à la préparation de teintures de cheveux permanentes, les substances odoriférantes ou les huiles de parfum contenant une préparation dépôt de la formule (I)

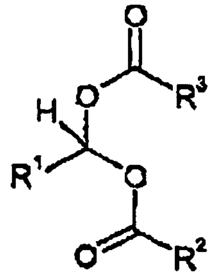

(I)

dans laquelle $R^1$, $R^2$et $R^3$ signifient indépendamment l'un de l'autre un radical organique avec 1 à 30 atomes de carbone et, après une hydrolyse ou une élimination enzymatique, le composé de la formule (I) libérant un aldéhyde et deux acides carboxyliques.

7. Utilisation selon la revendication 6, les substances odoriférantes ou l'huile de parfum contenant des extraits de matières premières naturelles, des hydrocarbures, des alcools aliphatiques, des cétones aliphatiques, des nitriles aliphatiques, des acides carboxyliques aliphatiques, des alcools terpéniques acycliques, des aldéhydes terpéniques acycliques, des aldéhydes terpéniques cycliques, des alcools cycliques, des alcools cycloaliphatiques, des éthers cycliques ou cycloaliphatiques, des cétones cycliques, des aldéhydes ou des cétones cycloaliphatiques, des esters d'alcools ou d'acides carboxyliques cycliques, des hydrocarbures aromatiques, des alcools araliphatiques, des esters d'alcools araliphatiques avec des acides carboxyliques aliphatiques, des aldéhydes, des cétones ou des acides carboxyliques aromatiques ou araliphatiques, des composés azotés aromatiques, des phénols, des phényléthers ou des phénylesters, des composés hétérocycliques ou des lactones.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9406441 A
- WO 0004009 A
- WO 0038616 A


### In der Beschreibung aufgeführte Nicht-Patentliteratur


- **A. Leo ; C. Hansch ; D. Elkins.** *Chem. Rev.,* 1971, vol. 71, 525-616
- **C. Hansch ; J.E. Quinlan ; G.L. Lawrence.** *J. Org. Chem.,* 1968, vol. 33, 347-350
- **S. Arctander.** Perfume and Flavor Materials. Selbstverlag, 1969, vol. I, II
- **K. Bauer ; D. Garbe ; H. Surburg.** Common Fragrance and Flavor Materials. Wiley-VCH, 1997
- **T.W. Greene ; P.G.M. Wuts.** Protective Groups in Organic Synthesis. John Wiley & Sons, Inc, 1991
- **Karimi, B ; Seradj, H. ; Ebrahimian, G.R.** *Synlett,* 2000, 623-624
- **Chandra, K.L. ; Saravanan, P. ; Singh, V.K.** *Synlett,* 2000, 359-360
- **Sydnes, L.K. ; Sandberg, M.** *Tetrahedron,* 1997, vol. 53, 12679-12690
- **Kumar, H.M.S ; Reddy, B.V.S. ; Reddy, P.T. ; Yadav, J.S.** *J. Chem. Res. (S),* 2000, 86-87
- **Jin, T.-S. ; Ma, Y.-R. ; Sun, X. ; Liang, D. ; Li, T.-S.** *J. Chem. Res. (S),* 2000, 96-97